# EUROPÄISCHE PATENTANMELDUNG

(11) **EP 0 932 040 A1**
(43) Veröffentlichungstag der Anmeldung: **28.07.1999**
(21) Anmeldenummer: 99100968.9
(22) Anmeldetag: 20.01.1999
(51) Int. Cl.: G01N 31/22, G01N 33/02

(54) **Verfahren zur Überprüfung des Verderblichkeitszustandes von Lebensmitteln**

(30) Priorität: 23.01.1998 DE 19802448
(71) Anmelder: Kleiböhmer, Guido, 45476 Mülheim a.d. Ruhr (DE); Matzik, Iduna Dr. rer. nat., 46049 Oberhausen (DE); Heiduk, Christoph Benedikt, 46119 Oberhausen (DE)
(72) Erfinder: Kleiböhmer, Guido, 45476 Mülheim a.d. Ruhr (DE); Matzik, Iduna Dr. rer. nat., 46049 Oberhausen (DE); Heiduk, Christoph Benedikt, 46119 Oberhausen (DE)

(57) **Zusammenfassung**

Analysetechnisch ist üblich, daß Lebensmittel, insbesondere Säfte, während und nach dem Produktionsprozeß oder nach der Abfüllung durch den Hersteller auf Ihre Güte mittels geeigneter Methoden und Indikatoren überprüft werden.
Verwendet werden dabei lebensmittelzugelassene oder natürliche Farbstoffe.
Nachteile sind, daß die herkömmlichen Indikatoren auf Folienbasis nicht durchsichtig sind und keine Farbindikatorsysteme existieren, die für den direkten Kontakt mit dem 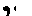 Verbraucher" des Lebensmittels (sog. 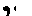 in-prozess-Kontrolle) geeignet und praktikabel sind.
Die Erfindung bezweckt durch Modifizierung der bekannten Trägermaterialien für Indikatorfarbstoffe sowie insbesondere durch Verwendung eines neuen Farbindikators, Mittel und Verfahren zur Überprüfung Verderblichkeitszustandes von Lebensmitteln, z. B. Fruchtsäften, auch in der Verpackung sowie für den Endverbraucher zu schaffen.
Diese Aufgabe wird gelöst mit einem Verfahren, das als Indikatorfarbstoff einen Farbstoff aus der Gruppe der Anthocyane verwendet, der einen signifikanten Farbumschlag im sauren Bereich (pH 2-4) zeigt. Der Indikatorfarbstoff geht dabei mit dem Polymerträgermaterial eine feste Verbindung ein.
Zudem wurden Mittel zur Durchführung des Verfahrens entwickelt.

## Beschreibung

Die Erfindung betrifft ein Verfahren und Mittel zur Überprüfung des Güte- bzw. Verderbliclikeitszustandes von Lebensmitteln, insbesondere Getränken, gemäß dem Oberbegriff des Anspruchs 1. sowie deren Verwendung.

Der Stand der Technik zur Analytik von Lebensmitteln oder Fruchtsäften ist erstens Kontrolle während und nach der Herstellung bzw. Abpackung sowie zweitens Stichprobenkontrolle nach der Abfüllung/Abpackung. Die Methoden sind Keimzahlbestimmung, Bakterienanfärbung mit Fluoreszenzfarbstoffen, wobei als Indikatoren dabei zum Beispiel Lackmus, Kristallviolett sowie Bromthymolblau verwendet werden. Weitere Methoden sind die Prüfung auf Pilze und Hefen mit 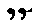 Pepton"/Glucose (Spezialindikator der Fa. Merck), die Prüfung auf Milchsäure mit p-Hydroxy-Diphenyl, die Prüfung auf Glucose mit Jodlösung, die Prüfung auf Gesamtzucker mit Kupfersulfat, Enzymen sowie Kupfertetramin, die Prüfung des Alkoholgehaltes und schließlich die Prüfung auf Gesamtsäure (Endpunkttitration). Als Literatur hierzu sei verwiesen auf D. Baumgart, Mikrobiologische Untersuchung von Lebensmitteln,
Zschaler, Mikrobiologische Untersuchungsmethoden sowie
Merck, Mikrobiologische Untersuchungsmethoden, 1968, Lebensmitteltechnologie. Die oben genannten Methoden sind sämtlich nur für den Produktions- und Abfüllprozeß anwendbar, nicht jedoch für den direkten Kontakt mit dem Verbraucher bzw. für eine sogenannte 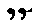 in-Prozess-Kontrolle".
Zum Stand der Technik hinsichtlich der verwendeten Indikatoren ist festzustellen, daß diese zunächst zur Verwendung bei der Prozeßoptimierung, für spezifische Indikatorpapiere/Messysteme oder zum Beispiel zur Feststellung des Reifestatusses von Obst hergestellt werden.
Eine Anwendung in Lebensmitteln bzw. Getränken, insbesondere in Fruchtsäften, bzw. Fruchtnektaren und Saftgetränken, ist nicht erkennbar. Die nach wie vor gebräuchlichen und lebensmittelzugelassenen Indikatoren sind zum Beispiel Eosin, ErythrosinB, Bromthymolblau sowie darüber hinaus einige Azofarbstoffe, die jedoch aufgrund ihrer immer wieder befürchteten cancerogenen Wirkung im Tierversuch nicht in Frage kommen.
An natürlichen Farbstoffen für Indikatoren gibt es nichts neues. Die gut untersuchten Lebensmittelfarbstoffe zeigen bis auf Chlorophyll, Brillantsäuregrün BS und Erythrosin keine Farbveränderung im in Frage kommenden pH-Bereich.
Hinsichtlich des Standes der Technik zur Verwendung von Farbstoffen aus der Gruppe der Anthocyane ist folgendes festzustellen: Anthocyane sind natürlich vorkommende Farbstoffe in nahezu allen Früchten, Blüten und Blättern. Die Anthocyane sind mit verantwortlich für die roten, blauen oder violetten Farbtöne. Es gibt rote und blaue Farbtypen. Die Farbe hängt ab von der Molekülstruktur, den umgebenden Eisen- und Aluminium-Ionen und dem pH-Wert.
Dies wird beschrieben bei Duruphty, André, et. al.: Indicateures colorés acido-basiques naturels, in: Bulletin de l'Union des Physiciens, 1987, 81 (694), S. 665-670.
Schon lange ist das Vorkommen der Anthocyane in Weinreben bekannt. Fast ebensolange werden diese Farbstoffe auch zur besseren Färbung von Weinen verwendet (heute nur noch in Italien). Sie sind sehr gut untersucht und als Lebensmittelfarbstoffe zugelassen.
1960 wurde dieser Farbstoff erstmals synthetisch hergestellt (E. Bayer). Ein bekannter deutscher Lieferant für diesen Farbstoff ist Simon und Werner; Hersteller ist Givaudan. In den letzten Jahren haben sich immer wieder Agrarwissenschaftler mit der Verwendung der Anthocyane beschäftigt. Diskutiert werden die Isolierung aus Blüten sowie der Zusatz zu Früchten oder Getränken.
Anwendung finden die Anthocyane als Farbstoffindikator für den Reifezustand von Früchten, wobei die Anthocyanegehalte in Früchten in Abhängigkeit vom Reifezustand gemessen werden, es gibt Untersuchungen zu Einflußparametern auf den Farbton (rot oder blau), zu Stabilitätsbeeinflussungen von Bier, Wein, Brandy durch Anthocyane, zum Verhalten der Anthocyane bei Lagerung von Früchten, Verfahren zur Färbung von Seide oder Cellulose mit Anthocyanen.
Dazu wird z. B. in DE 4445624 A1 Stadler, Christian, die Herstellung eines Indikatorpapiers bestehend aus Filterpapier und einem Extrakt der Farbstoffe von Stiefmütterchen oder Rettichwurzeln beschrieben.

Bekannt ist auch eine Anmeldung zur Farbveränderung von Mundhygieneprodukten, wobei Anthocyane zur Farbveränderung benutzt werden (R. M. Buch, WO 9629.047, 26.09.1996).
Hinsichtlich des Standes der Technik der Trägermaterialien der Indikatoren ist festzustellen, daß es Indikatorsysteme auf Basis Polymere gibt. Als Beispiel sei hier genannt die Indikatorstäbchen auf Cellulose-Basis der Fa. Merck. Im medizinischdiagnostischen Bereich ist es aber auch üblich, spezielle Folien- Indikatoren zu verwenden.
Üblich sind hier Cellulose, Co-Polymer, Polyester sowie Polysiloxane ( 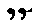 Silicone") PDMS.
Das Prinzip sieht dabei wie im folgenden beschrieben aus:
Ein Papierstäbchen (Cellulose) wird mit einem Gemisch aus speziellen Farbindikatoren getränkt, wobei darüber eine Folie angesiedelt werden kann, die aber nur zum Schutz vor Ausbluten oder Altern dient.
Alternativ ist es Stand der Technik daß auf ein Grundgerüst aus Cellulose oder Polyester eine Testzone (Folie + Indikatorgemisch) aufgesiedelt wird.
Bekannt ist zudem, daß als Trägermaterial für Farbstoffe das Ionomer verwendet werden kann. Das Ionomer ist ein Co-Polymer aus Ethylen und Acrylsäure, in dem einige Wasserstoff-Ionen der Säuregruppen durch Natrium oder Zink ersetzt wurden. So ist bei dieser Folie die Oberfläche nicht, wie üblich, ungeladen, sondern enthält zu 10% Ionen. Dadurch erhält diese Folie besondere Eigenschaften, sie kann als Ionentauscher fungieren, sie kann beschichtet werden (z. B. mit Farbe) und sie geht äußerst stabile ionische Bindungen ein, wie zum Beispiel zu Aluminiumbeschichtungen, die in Getränkepackungen Verwendung finden, um als Gasbarriere zu dienen, um Sauerstoffeintritt und damit ein höheres Verderbs-Risiko zu vermeiden.
Hergestellt werden diese Folien-Verbunde heute durch Ko-Extrusion, daß heißt durch gleichzeitiges Ausgießen oder Herausspritzen von verschiedenen geschmolzenen Kunststoffen durch eine Düse; beim Abkühlen und Strecken entsteht der Verbund.

Ein entscheidender Nachteil ist bei diesen herkömmlichen Indikatoren auf Folienbasis, daß es keine durchsichtigen Indikatorfolien gibt.

Bekannt ist weiter, daß Tests des Lagerverhaltens von Lebensmitteln mittels Indikatorpapieren, -folien sowie -membranen durchgeführt werden. Zu erwähnen ist hier insbesondere 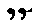 Multicromic polymers for food packaging" (Seb. Kanakkanatt, 07.03.196, PCT Int. Appl. WO 9606.643, US Appl. 298465, 30.08.1994) Das Prinzip ist hierbei, daß ein Teil der Verpackung auf Basis Polystyrol plus Farbindikator reagiert auf Veränderungen des Lebensmittels infolge unsachgemäßer Lagerung, das heißt Einfluß von Licht, NO2 oder Hitze. Die benutzten Indikatoren sind herkömmliche, die Basis ist Polystyrol und der Test betrifft nur die unsachgemäße Lagerung, nicht aber das 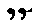 normale" Verderben.
Bekannt ist auch, daß zur Feststellung und Anzeige des Bakterienwachstums bei Lebensmitteln Lipid-Membranen verwendet werden. Hingewiesen sei in diesem Zusammenhang auf 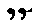 Food freshness indicators containing filter paper, lipid membranes, and pigments" (JP 0815.251, 19.01.1996). Prinzip ist hier, daß die Lipid-Membranen durch Bakterien zerstört werden. Dabei wird der Farbstoff, der in der Membran enthalten ist, freigesetzt und sichtbar. Das ganze geschieht zum Beispiel mit Stärke auf Filterpapier plus Sojalecithin plus Spirulina Pigment, umhüllt mit einer transparenten Folie. Ungeeignet ist dieses Prinzip für Getränke. Auch werden wieder herkömmliche Farbindikatoren verwendet.
Bekannt sind weiterhin Methoden zur Messung der Stabilität von Fruchtsäften, die jedoch alle die Lagerstabilität oder das kosmetische Erscheinungsbild (Ausflockung, Farbe, Viskosität) des Saftes betreffen. Bekannt sind auch Methoden zur Analytik von Fruchtsäften, bzw. Fruchtnektaren oder Saftgetränken, jedoch findet sich nicht eine, die zur Kontrolle des Gütezustandes in der (End-) Verpackung entwickelt oder geeignet sind. Es handelt sich vielmehr um Methoden zur Prozeßoptimierung bei der Herstellung von Getränken.

Jegliche oben genannten Methoden der Analytik von Lebensmitteln, insbesondere von Fruchtsaftgetränken, bzw. Fruchtnektaren oder Saftgetränken, sind ungeeignet für den Gebrauch durch den Endverbraucher. Es existiert nicht eine Methode zur Analytik in der Verpackung. Die verwendeten Trägermaterialien der Indikatoren sind nicht durchsichtig, was ihre Verwendung einschränkt. Als Farbindikatoren werden nur die oben genannten verwendet.

Die Erfindung bezweckt durch Modifizierung der bekannten Trägermaterialien für Farbindikatoren sowie insbesondere durch Verwendung eines neuen Farbindikators, Geräte und Verfahren zur Überprüfung des Gütezustandes/Verderblichkeitszustandes von Lebensmitteln, insbesondere Fruchtsäften, bzw. Fruchtnektaren oder Saftgetränken, in der Verpackung und insbesondere für den Endverbraucher zu schaffen. Eine Möglichkeit zur einfachen Überprüfung für den Endverbraucher gibt es derzeit nicht, obwohl dies erforderlich scheint, da die Kontamination des Lebensmittels in den meisten Fällen erst nach Öffnen der Verpackung eintritt. Hinsichtlich der Haltbarkeit des Lebensmittels in ungeöffneten Zustand gibt das Mindesthaltbarkeitsdatum, das auf der Verpackung angebracht sein muß, dem Verbraucher einen meist zuverlässigen Hinweis. Nach dem Öffnen der Verpackung hat der Verbraucher keine einfache und praktikable Möglichkeit, den Zustand des Lebensmittels zu überprüfen.
Die Modifizierung ermöglicht es dem Verbraucher durch eine Farbveränderung des Indikators eine ständige Kontrolle über den Gütezustand des Getränks zu erhalten.

Diese Aufgabe wird erfindungsgemäß mit einem Verfahren gelöst, das als Indikatorfarbstoff einen Farbstoff aus der Gruppe der Anthocyane benutzt. Das Trägermaterial des Indikators besteht aus oder ist beschichtet mit einem Polymer, das im sauren pH-Bereich (2 - 4) beständig ist, vorzugsweise aus den Materialien Polyester (Polyethylenterephtalat 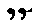 PET", PETG), Polymethylmethacrylat 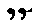 PMMA", Ionomer, Ethylenvinylacetat 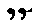 EVA", Säurecopolymer.
Dieses Polymer ist belegt mit einem Indikatorfarbstoff, vorzugsweise aus der Gruppe der Anthocyane, der einen signifikanten Farbumschlag in sauren Bereich (pH 2 - 4) zeigt.
Der Indikatorfarbstoff und das Polymer gehen eine feste Verbindung ein, die in wässriger Lösung nicht mehr reversibel ist. Dieser neuartige Indikator zeigt hingegen in wässriger Lösung in Abhängigkeit von den darin vorgehenden chemischen Reaktionen einen Farbumschlag. Das Polymer kann in flüssiger oder fester Form vorliegen, z. B. als Folie, Granulat, Pulver oder Pellets.

Die Indikatorfolie z. B. hat im ungeöffneten, frischen Zustandes eines Fruchtsaftgetränkes eine rote Farbe. Sobald nun der Zustand der Verderblichkeit des Fruchtsaftgetränkes eintritt, ändert sich der Farbton: Die 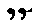 Indikatorfolie" wird zunächst farblos, bis zu einem Farbumschlag nach blau-violett bei völliger Ungenießbarkeit.
Für den 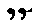 Verbraucher" des Getränkes wird durch die Farbveränderung des Stabes der Verderblichkeitsgrad, bzw. die Ungenießbarkeit sichtbar. Der Gütezustand, bzw. der Verderblichkeitszustand wird somit in dem Behälter, bzw. der Verpackung sichtbar.

Die Folie, im weiteren bezeichnet als 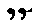 Indikatorfolie", wird in einem Herstellgang des Produktionsprozesses mit einer wäßrigen Lösung eines Anthocyans, vorzugsweise eines Vertreters der rotfärbenden Gruppe, zum Beispiel 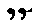 Anthocyan LMF" der Fa. Givaudan, versetzt. Der Farbstoff zieht irreversibel auf die Folie auf, bzw. kann zur besseren Haftung noch alkalisiert werden.

Die Farbveränderung erklärt sich dadurch, daß beim Verderben des Getränkes (Saftes) der pH-Wert aufgrund der Abnahme des Gehaltes der Gesamtsäure leicht ansteigt. Darauf reagieren die erfindungsgemäß einsetzten Anthocyane mit einem Farbumschlag: Solange das Getränk (Saft) frisch ist, zeigt sich eine rote, durchscheinende Farbe.
Beim Verderben des Getränkes, also beim Anstieg des pH-Wertes, verändert sich die Farbe zunächst derart, daß die Farbe verblaßt und schließlich bei völliger Ungenießbarkeit des Getränkes nach blau-violett umschlägt.
Somit ist für den Verbraucher durch die Farbumschläge leicht erkennbar, ob das Getränk oder der Saft frisch oder verdorben, das heißt ungenießbar ist.

Ein Mittel gemäß Anspruch 2. und 3. zur Durchführung des Verfahrens nach Anspruch 1. zeigt in der Zeichnung
Fig. 1.

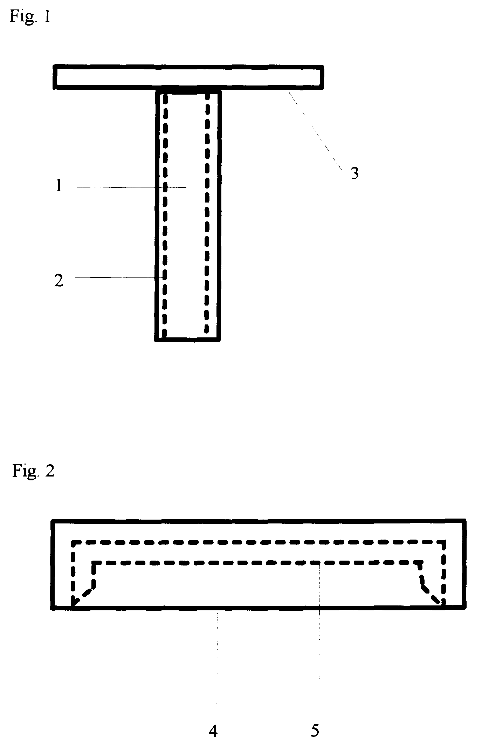

Fig. 1 zeigt zur Durchführung des Verfahrens nach Anspruch 1. einen dreidimensionalen Gegenstand, vorzugsweise einem Stab **1**, der am Verschlußsystem **3** des Behälters, bzw. der Verpackung angebracht ist und in die Lösung/das Getränk eintaucht.

Der Stab **1** besteht aus oder ist beschichtet mit einer Folie **2**, die im sauren pH-Bereich (2-5) beständig ist, vorzugsweise aus den Materialien Polyester (Polyethylenterephtalat 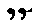 PET", PETG), Polymethylmethacrylat 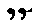 PMMA", Ionomer, Ethylenvinylacetat 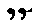 EVA", Säurecopolymer.
Diese Folie **2** ist beladen mit einem Indikatorfarbstoff, vorzugsweise aus der Gruppe der Anthocyane, der einen signifikanten Farbumschlag in sauren Bereich (pH 2 - 4) zeigt.

Am Verschlußsystem **3** des Behälters angebracht, hat der mit der Indikatorfolie versehene Stab **1** zum Zeitpunkt des ungeöffneten, frischen Zustandes des Getränkes, eine rote Farbe. Sobald nun der Zustand der Verderblichkeit des Getränkes eintritt, ändert sich der Farbton: Der 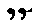 Indikatorstab" **1** wird zunächst farblos, bis zu einem Farbumschlag nach blau-violett bei völliger Ungenießbarkeit.
Für den 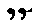 Verbraucher" des Getränkes wird durch die Farbveränderung des Stabes **1** der Verderblichkeitsgrad, bzw. die Ungenießbarkeit sichtbar. Der Gütezustand, bzw. der Verderblichkeitszustand wird somit in dem Behälter, bzw. der Verpackung sichtbar.
Die Folie **2,** im weiteren bezeichnet als 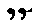 Indikatorfolie", wird in einem Herstellgang des Produktionsprozesses mit einer wäßrigen Lösung eines Anthocyans, vorzugsweise eines Vertreters der rotfärbenden Gruppe, zum Beispiel 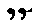 Anthocyan LMF" der Fa. Givaudan, versetzt. Der Farbstoff zieht irreversibel auf die Folie **2** auf bzw. kann zur besseren Haftung noch alkalisiert werden.
Die Folie **2** kann nun auf ein geeignetes Trägermaterial, zum Beispiel Polyethylen 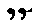 PE", auf den oben beschriebenen Stab **1** aufgebracht werden.

Den Querschnitt eines Mittels gemäß Anspruch 2. und 4. zur Durchführung des Verfahrens nach Anspruch 1. zeigt in der Zeichnung
Fig. 2.
Fig. 2 zeigt zur Durchführung des Verfahrens nach Anspruch 1. ein Verschlußsystem **4** (Deckel, Verschraubung), dessen Innendichtung **5** aus einer speziellen Folie besteht bzw. mit einer solchen beschichtet ist, vorzugsweise aus einem der Materialien Polyester, PMMA, Ionomer, EVA oder Säurecopolymer, beladen mit einem Indikatorfarbstoff aus der Gruppé der Anthocyane.
Der Indikatorfarbstoff zeit einen signifikanten Farbumschlag im sauren Bereich (pH-Wert 2-4).

Dazu wird die Folie **5**, im weiteren bezeichnet als 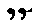 Indikatorfolie", in einem Herstellgang des Produktionsprozesses mit einer wäßrigen Lösung eines Anthocyans, vorzugsweise eines Vertreters der rotfärbenden Gruppe, zum Beispiel 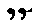 Anthocyan LMF" der Fa. Givaudan, versetzt. Der Farbstoff zieht irreversibel auf die Folie **5** auf bzw. kann zur besseren Haftung noch alkalisiert werden.

Am Verschiußsystem **4** des Behälters angebracht, hat die mit der Indikatorfolie **5** versehene Innendichtung **5** zum Zeitpunkt des ungeöffneten, frischen Zustandes des Getränkes eine rote Farbe.

Sobald nun der Zustand der Verderblichkeit des Getränkes eintritt, ändert sich der Farbton: Die 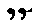 Indikatordichtung" **5** wird zunächst farblos, bis zu einem Farbumschlag nach blau-violett bei völliger Ungenießbarkeit.
Für den 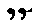 Verbraucher" des Getränkes wird durch die Farbveränderung der Dichtung der Verderblichkeitsgrad, bzw. die Ungenießbarkeit sichtbar. Der Gütezustand, bzw. der Verderblichkeitszustand wird somit in dem Behälter, bzw. der Verpackung sichtbar.

Der erfindungsgemäße Gegenstand gemäß Anspruch 5. betrifft eine Modifikation von Getränkeverpackungen, vorzugsweise Tetrapacks, On-packs oder brick-packs, dadurch gekennzeichnet, daß in das Verpackungsmaterial ein Fenster eingelassen ist, bestehend aus einem Polymer, vorzugsweise einer Ionomer-Folie des Verpackungsverbundmaterials, wobei als Indikatorfarbstoff ein Farbstoff aus der Gruppe der Anthocyane verwendet wird.

Fig. 3
zeigt einen Querschnitt durch den Verbundaufbau einer Geträrkeverpackung am Beispiel eines Tetra-packs, in dem das Fenster **6** zu sehen ist.

Die Modifikation gestaltet sich wie folgt:
In das Verpackungsmaterial ist ein Fenster **6** eingelassen, bestehend aus einer Ionomer-Folie 7 des Verpackungsverbundmaterials, daß heißt ein Kleiner Ausschnitt der Ionomer-Innenfolie 7, beispielsweise von der Größe 1 x 1 cm, ist freigelegt durch das Entfernen der übrigen Schichten PE **8,** Aluminium **10** und Papier **9** sowie der Innenschicht **11**, **8**. Übrig bleibt an dieser Stelle eine hochtransparente Folie 7 Diese Folie 7 wird - entweder vor Herstellung der Faltschachtel oder im Anschluß daran - mit einem Indikatorfarbstoff aus der Gruppe der Anthocyane beschichtet. Dazu wird die Folie 7, im weiteren bezeichnet als 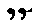 Indikatorfolie" 7, in einem Herstellgang des Produktionsprozesses mit einer wäßrigen Lösung eines Anthocyans, vorzugsweise eines Vertreters der rotfärbenden Gruppe, zum Beispiel 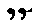 Anthocyan LMF" der Fa. Givaudan, versetzt. Der Farbstoff zieht irreversibel auf die Folie 7 auf, bzw. kann zur besseren Haltung noch alkalisiert werden.
Dieser Indikatorfarbstoff zeigt einen signifikanten Farbumschlag von Rot nach Blau im pH-Bereich 2-4.
Diese Anordnung hat den Vorteil, daß der Verbraucher eine ständige Kontrolle über den Gütezustandes des Getränkes hat. Solange das Getränk (bzw. Saft, Nektar, Saftgetränk) frisch ist, sieht der Verbraucher an der Stelle des Fensters **6** eine rote, durchscheinende Farbe. Beim Verderben des Getränkes verändert sich die Farbe in dem Fenster 6 aufgrund des eingesetzten Indikatorfarbstoffes aus der Gruppe der Anthocyane zunächst derart, daß die Farbe verblaßt und schließlich nach blau-violett umschlägt.
Zum Schutz vor äußerer Einwirkung wie Licht, Sauerstoff oder mechanischer Zerstörung ist das Fenster **6** mit einer 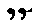 Blisterfolie" **12,** zum Beispiel aus Aluminium abgedeckt (Prinzip der Trinkhalmabdeckung bzw. der Zahnpasta- oder Haarfärbetuben).
Erst bei der In-Gebrauch-Nahme durch den Verbraucher wird die Blisterfolie **12** entfernt und so das Fenster **6** freigelegt.
**13** zeigt die Außenseite des Verbundmaterials.

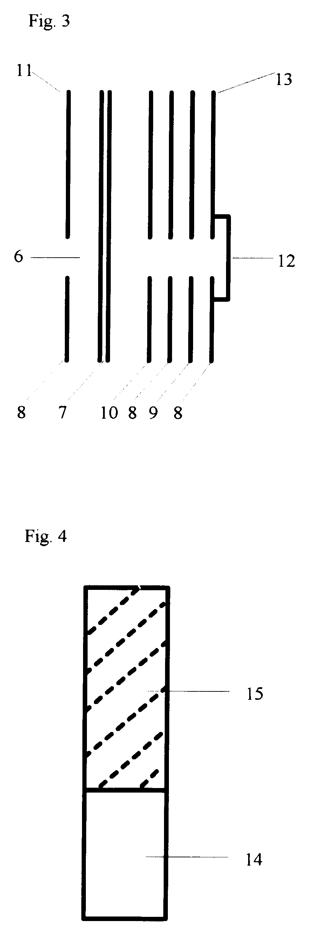

Fig. 4 zeigt in der Aufsicht ein Mittel gemäß Anspruch 2. und 6. zur Durchführung des Verfahrens gemäß Anspruch 1.

Der erfindungsgemäße Gegenstand bezeichnet ein Meßstreifen **14,** wobei als Indikatorfarbstoff ein Farbstoff aus der Gruppe der Anthocyane verwendet wird, der einen signifikanten Farbumschlag in sauren Bereich (pH 2-4) zeigt.
Der Meßstreifen **14** besteht aus oder ist beschichtet mit einem Polymer, insbesondere einer Folie, die im sauren pH-Bereich (2 - 4) beständig ist, vorzugsweise aus den Materialien Polyester (Polyethylenterephtalat 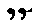 PET", PETG), Polymethylmethacrylat 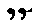 PMMA", Ionomer, Ethylenvinylacetat 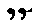 EVA", Säurecopolymer.

Diese Folie ist beladen mit einem Indikatorfarbstoff **15**, vorzugsweise aus der Gruppe der Anthocyane, der einen signifikanten Farbumschlag in sauren Bereich (pH 2 - 4) zeigt.

Der mit der Indikatorfolie versehene Meßstreifen kann in ein Getränk oder Saft oder Lebensmittel eingetaucht werden und zeigt zum Zeitpunkt des frischen Zustandes des Getränkes, Saftes oder Lebensmittels eine rote Farbe. Falls nun der Zustand der Verderblichkeit des Getränkes, Saftes oder Lebensmittels eintritt, ändert sich der Farbton: Der 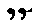 Indikatormeßstreifen" wird zunächst farblos, bis zu einem Farbumschlag nach blau-violett bei völliger Ungenießbarkeit.
Für den 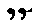 Verbraucher" des Getränkes, Saftes oder Lebensmittels wird durch die Farbveränderung des Indikatormeßstreifens der Verderblichkeitsgrad, bzw. die Ungenießbarkeit sichtbar. Der Gütezustand, bzw. der Verderblichkeitszustand wird somit an dem Meßstreifen sichtbar.
Die verwendete Folie wird in einem Herstellgang des Produktionsprozesses mit einer wäßrigen Lösung eines Anthocyans, vorzugsweise eines Vertreters der rotfärbenden Gruppe, zum Beispiel 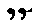 Anthocyan LMF" der Fa. Givaudan, versetzt. Der Farbstoff zieht irreversibel auf die Folie auf bzw. kann zur besseren Haftung noch alkalisiert werden.
Die Folie kann nun auf ein geeignetes Trägermaterial, zum Beispiel Polyethylen 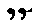 PE", auf den oben beschriebenen Meßstreifen aufgebracht werden.

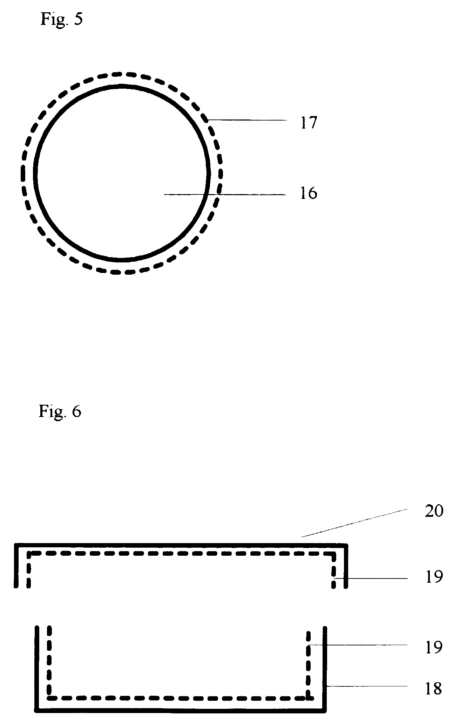

Fig. 5 zeigt im Querschnitt eine Ausführungsform des Mittels nach Anspruch 2. und 7. zur Durchführung des Verfahrens nach Anspruch 1.
Der erfindungsgemäße Gegenstand bezeichnet einen dreidimensionalen Gegenstand, vorzugsweise eine Kugel **16** oder einen Quader, der bei dem Verzehr eines Getränkes, Saftes oder Lebensmittels in dieses hineingegeben wird, schwimmt und dadurch gekennzeichnet ist, daß als Indikatorfarbstoff ein Farbstoff aus der Gruppe der Anthocyane verwendet wird, der einen signifikanten Farbumschlag in sauren Bereich (pH 2-4) zeigt.

Der Indikatorgegenstand **16** besteht aus oder ist beschichtet mit einer Folie, die im sauren pH-Bereich (2 - 4) beständig ist, vorzugsweise aus den Materialien Polyester (Polyethylenterephtalat 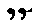 PET", PETG), Polymethylmethacrylat 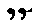 PMMA", Ionomer, Ethylenvinylacetat 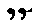 EVA", Säurecopolymer.
Diese Folie ist beladen mit einem Indikatorfarbstoff **17**, vorzugsweise aus der Gruppe der Anthocyane, der einen signifikanten Farbumschlag in sauren Bereich (pH 2 - 4) zeigt.

Der mit der Indikatorfolie versehene Indikatorgegenstand kann in ein Getränk oder Saft oder Lebensmittel eingetaucht werden und zeigt zum Zeitpunkt des frischen Zustandes des Getränkes, Saftes oder Lebensmittels eine rote Farbe. Falls nun der Zustand der Verderblichkeit des Getränkes, Saftes oder Lebensmittels eintritt, ändert sich der Farbton: Der 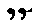 Indikatorgegenstand" wird zunächst farblos, bis zu einem Farbumschlag nach blau-violett bei völliger Ungenießbarkeit.
Für den 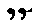 Verbraucher" des Getränkes, Saftes oder Lebensmittels wird durch die Farbveränderung des Indikatorgegenstandes der Verderblichkeitsgrad, bzw. die Ungenießbarkeit sichtbar. Der Gütezustand, bzw. der Verderblichkeitszustand wird somit an dem Indikatorgegenstand sichtbar.
Die verwendete Folie, wird in einem Herstellgang des Produktionsprozesses mit einer wäßrigen Lösung eines Anthocyans, vorzugsweise eines Vertreters der rotfärbenden Gruppe, zum Beispiel 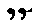 Anthocyan LMF" der Fa. Givaudan, versetzt. Der Farbstoff zieht irreversibel auf die Folie auf bzw. kann zur besseren Haftung noch alkalisiert werden.

Die Folie kann nun auf ein geeignetes Trägermaterial, zum Beispiel Polyethylen 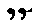 PE", auf den oben beschriebenen Gegenstand aufgebracht werden.

Fig. 6 zeigt den Querschnitt eines Mittels nach Anspruch 2. und 8.. zur Durchführung des Verfahrens nach Anspruch 1.
Der erfindungsgemäße Gegenstand bezeichnet einen wiederverschließbaren Vorratsbehälter **18**, vorzugsweise eine Dose (wie zum Beispiel eine Tupper-Dose), dadurch gekennzeichnet, daß der Behälter **18** selbst oder dessen Verschluß **20** als Indikator ausgestaltet ist und daß als Indikatorfarbstoff ein Farbstoff aus der Gruppe der Anthocyane verwendet wird, der einen signifikanten Farbumschlag in sauren Bereich (pH 2-4) zeigt.
Der Indikatorvorratsbehälter **18** oder dessen Verschluß **20** oder beides besteht aus oder ist beschichtet mit einer Folie **19**, die im sauren pH-Bereich (2 - 4) beständig ist, vorzugsweise aus den Materialien Polyester (Polyethylenterephtalat 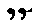 PET", PETG), Polymethylmethacrylat 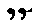 PMMA", Ionomer, Ethylenvinylacetat 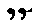 EVA", Säurecopolymer.
Diese Folie **19** ist beladen mit einem Indikatorfarbstoff vorzugsweise aus der Gruppe der Anthocyane, der einen signifikanten Farbumschlag in sauren Bereich (pH 2 - 4) zeigt.

In den mit der Indikatorfolie **19** versehenen Indikatorvorratsbehälter **18** kann in ein Getränk oder Saft oder Lebensmittel eingelagert werden. Der Indikatotvorratsbehälter **18** zeigt zum Zeitpunkt des frischen Zustandes des Getränkes, Saftes oder Lebensmittels eine rote Farbe. Falls nun der Zustand der Verderblichkeit des Getränkes, Saftes oder Lebensmittels eintritt, ändert sich der Farbton: Der 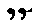 Indikatorvorratsbehälter" **18** wird zunächst farblos, bis zu einem Farbumschlag nach blau-violett bei völliger Ungenießbarkeit.
Für den 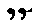 Verbraucher" des Getränkes, Saftes oder Lebensmittels wird durch die Farbveränderung des Indikatorvorratsbehälters **18** der Verderblichkeitsgrad, bzw. die Ungenießbarkeit des eingelagerten Getränkes, Saftes oder Lebensmittels sichtbar. Der Gütezustand, bzw. der Verderblichkeitszustand wird somit an dem Indikatorvorratsbehälter **18** oder dessen Verschluß **20** sichtbar.
Die verwendete Folie wird in einem Herstellgang des Produktionsprozesses mit einer wäßrigen Lösung eines Anthocyans, vorzugsweise eines Vertreters der rotfärbenden Gruppe, zum Beispiel ,,Anthocyan LMF" der Fa. Givaudan, versetst. Der Farbstoff zieht irreversibel auf die Folie auf, bzw. kann zur besseren Haftung noch alkalisiert werden.
Die Folie kann nun auf ein geeignetes Trägermaterial, zum Beispiel Polyethylen 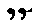 PE", auf den oben beschriebenen Vorratsbehälter **18** aufgebracht werden.
Gleiches gilt für den Verschluß **20** des Behälters.
Er kann auf die gleiche Weise hergestellt, beschichtet und als Indikatorverschluß verwendet werden.

Gewerblich anwendbar sind sowohl die Verfahren nach Anspruch 1.und Anspruch 9. sowie die beschriebenen Mittel nach den Ansprüchen 2. bis 8.
Die Recherche hat ergeben, daß durchsichtige Indikatorfolien z. B. zur Bestimmung des pH-Wertes auf dem Markt nicht verfügbar sind. Vorteile bieten durchsichtige Indikatorfolien vor allem im medizinisch-diagnostischen Bereich. Das Verfahren nach Anspruch 1. sowie die Mittel sind vor allem für die Getränkeindustrie interessant. Dem Verbraucher wird die Möglichkeit gegeben, auf einfache Art und Weise den Güte- bzw. den Verderblichkeitszustandes des gekauften Produktes zu überprüfen. Aufgrund des in den letzten Jahren erheblich gesteigerten Gesundheitsbewußtseins der Verbraucher kann sich ein solches Produkt am Markt durchsetzen. Die Herstellungskosten sind gering und die Anwendung ist unkompliziert.
Vorstellbar sind ebenso z. B. mit dem Indikatorfarbstoff beschichtete Lebensmittelverpackungen wie Aluniniumdosen, die nach dem Öffnen bzw. Aufreißen die Farbveränderung und damit den Güte- bzw. Verderblichkeitszustand des Lebensmittels.

## Patentansprüche

1. Verfahren zur Überprüfung des Verderblichkeitszustandes von Lebensmitteln, insbesondere Getränken, mittels eine speziellen Polymeren, insbesondere aus dem Bereich Ionomer, und eines Indikatorfarbstoffes, **dadurch gekennzeichnet**, daß der Indikator ein Farbstoff aus der Gruppe der Anthocyane ist, der einen Farbumschlag im sauren Bereich (pH 2 - 4) zeigt, der Indikatorfarbstoff mit dem Polymer eine feste Verbindung eingeht und wobei das Polymer mit der zu untersuchenden Probe in Kontakt gebracht wird.

2. Mittel zur Überprüfung des Verderblichkeitszustandes von Lebensmitteln, insbesondere Fruchtsaft oder Fruchtsaftgetränken, bestehend aus einem einen Indikator aufweisenden Polymer, **dadurch gekennzeichnet**, daß der Indikator ein Farbstoffaus der Gruppe der Anthocyane ist, der einen Farbumschlag im sauren Bereich (pH 2 - 4) zeigt und mit dem Polymer eine feste Verbindung eingeht.

3. Mittel nach Anspruch 2., insbesondere ein Stab, wobei dieses Gerät besteht aus oder beschichtet ist mit einem Polymer, vorzugsweise aus den Materialien Polyester (Polyethylenterephtalat 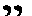 PET", PETG), Polymethylmethacrylat 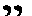 PMMA", Ionomer, Ethylenvinylacetat 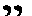 EVA", Säurecopolymer, **dadurch gekennzeichnet**, daß es am Verschußsystem eines Behälters oder einer Verpackung angebracht ist und in die Lösung, das Lebensmittel oder das Getränk eintaucht.

4. Mittel nach Anspruch 2. oder 3. insbesondere ein Verschlußsystem, ein Deckel oder eine Verschraubung, das als Innendichtung benutzt wird oder mit einer Innendichtung versehen ist, wobei das Gerät oder die Innendichtung besteht aus oder beschichtet ist mit einem Polymer, **dadurch gekennzeichnet,** daß das Mittel oder deren Innendichtung beschichtet ist mit dem Indikatorfarbstoff.

5. Mittel nach Anspruch 2. oder 3. für Getränkeverpackungen, vorzugsweise Tetra-packs, On-packs oder Brick-packs, **dadurch gekennzeichnet**, daß in das Verpackungsmaterial ein Fenster eingelassen ist, bestehend aus einem Polymer, insbesondere einer Ionomerfolie, des Verpackungsverbundmaterials, wobei das Polymer mit dem Indikatorfarbstoff versehen ist.

6. Mittel nach Anspruch 2. oder 3., insbesondere ein Meßstreifen, dadurch gekennzeichnet, daß der Meßstreifen mit dem Indikatorfarbstoff versehen ist.

7. Mittel nach Anspruch 2. oder 3., vorzugsweise eine Kugel oder ein Quader, bestehend aus oder beschichtet mit einem Polymer, insbesondere einer Ionomerfolie, das in ein Getränk, Saft oder Lebensmittel hineingegeben wird, schwimmt und **dadurch gekennzeichnet** ist, daß es mit dem Indikatorfarbstoff versehen oder beschichtet ist.

8. Mittel nach Anspruch 2. oder 3., insbesondere ein wiederverschließbarer Vorratsbehälter, vorzugsweise eine Dose, oder ein Verschluß für einen Vorratsbehälter, bestehend aus oder beschichtet mit einem Polymer, insbesondere einer Ionomerfolie, **dadurch gekennzeichnet**, daß das Mittel oder dessen Verschluß als Indikator ausgestaltet ist und mit dem Indikatorfarbstoff versehen oder beschichtet ist.

9. Verfahren zur Überprüfung des pH-Wertes von Lösungen mittels eines speziellen Polymeren, das im sauren pH-Bereich beständig ist (pH 2-4) und eines Indikatorfarbstoffes, wobei das Polymer mit dem Farbstoff eine feste Verbindung eingeht, **dadurch gekennzeichnet**, daß zur Überprüfung eine Kombination aus einem Polymer und einem Indikatorfarbstoff aus der Gruppe der Anthocyane verwendet wird, der einen Farbumschlag im sauren Bereich (pH 2 - 4) zeigt.
